# EUROPEAN PATENT APPLICATION

(11) **EP 4 023 762 A1**
(43) Date of publication of application: **06.07.2022**
(21) Application number: 20217671.5
(22) Date of filing: 29.12.2020
(51) Int. Cl.: C12P 19/02, C12P 19/14, C12P 7/10, C12P 7/40, C12N 1/38, C12M 1/00

(54) **METHOD AND SYSTEM FOR REDUCING INHIBITORY SUBSTANCES OF A LIGNOCELLULOSIC BIOMASS-BASED MATERIAL**

(71) Applicant: Sekab E-Technology AB, 891 26 Örnsköldsvik (SE)
(72) Inventor: JÖNSSON, Leif, 907 50 Umeå (SE); ALRIKSSON, Björn, 891 96 Arnäsvall (SE); ILANIDIS, Dimitrios, 891 61 Örnsköldsvik (SE)
(74) Representative: Kransell & Wennborg KB

(57) **Abstract**

The present invention relates to a method for reducing inhibitory substances of a lignocellulosic biomass-based material. The method comprises: pretreating a lignocellulosic biomass material in a pretreatment arrangement and subjecting the lignocellulosic biomass material to an elevated temperature and pressure in a reactor vessel; hydrolysing the pretreated biomass material in a hydrolysis unit; and adding at least one reducing agent selected from sulfur oxyanions and sulfhydryl reagents to the lignocellulosic biomass-based material downstream of the reactor vessel for reducing inhibitory substances. The concentration of the reducing agent is adapted based on the temperature of the material where it is added, and/or the reducing agent is added to the material having a temperature of T °C, T °C being more than 50 °C.

## Description

### TECHNICAL FIELD

The present invention relates to a method and a system for reducing inhibitory substances of a lignocellulosic biomass-based material. The invention further relates to a novel use of at least one reducing agent for decreasing the enzymatic hydrolysis inhibitory properties, and/or decreasing the microorganisms' fermentation inhibitory properties of a pretreated lignocellulosic biomass-based material. The method is inter alia useful in the manufacture of a fermentation product, such as ethanol, from the lignocellulosic biomass material.

### BACKGROUND

Lignocellulosic residues from forestry are attractive as feedstocks for the production of green chemicals and fuels, since they are abundant, relatively inexpensive, and not used for food. Lignocellulose consists mainly of lignin and two classes of polysaccharides, cellulose and hemicellulose. The polysaccharides can be hydrolysed to sugars and converted to various fermentation products, e.g. bio-alcohols, by means of fermenting microorganisms, such as Saccharomyces cerevisiae.

The hydrolysis of cellulose is typically preceded by a pretreatment, in which the hemicellulose is degraded and the cellulose is made increasingly accessible to cellulolytic enzymes or acidic hydrolysis. Enzymatic hydrolysis of lignocellulosic materials is considered the most promising method to obtain a high yield of glucose from cellulose. By using enzymatic hydrolysis, hydrolysis and fermentation can be performed simultaneously in a simultaneous saccharification and fermentation (SSF) process or in a consolidated bioprocess (CBP). Alternatively, separate hydrolysis and fermentation (SHF) can be used, a process configuration that may also include enzyme-based hydrolysis of the cellulose.

In industrial processes for converting lignocellulosic biomass to fermentation products, such as cellulosic ethanol, the pretreatment is considered an important part of the process as it affects downstream processes and determines the ultimate sugar yields. A variety of pretreatment processes exist, many of which rely on high temperature treatments and high pressures. To obtain high yields of sugars from lignocellulosic substrates, dilute acid hydrolysis pretreatment and/or steam pretreatment with acid catalysts are considered appropriate pretreatment methods. The pretreatment process is complex and involves many reactions and side-reactions. Such reactions may result in the formation of various by-products, which may be inhibitory to downstream processes, such as enzymatic hydrolysis and fermentation.

Various measures for improving the enzymatic hydrolysis, and dealing with negative side-effects, are known. For example, the addition of surfactants has been considered for improving enzymatic saccharification of cellulosic substrates. Surfactants probably prevent unproductive binding of enzymes to complex lignocellulosic substrates, such as pretreated wood. The economic benefit of adding surfactants to reaction mixtures intended for production of yield-sensitive low-value-added products such as liquid biofuels has, however, been questioned. Moreover, the addition of a reducing agent to reduce the inhibitory properties during enzymatic hydrolysis, is known.

As the enzymatic hydrolysis, and in particular the addition of enzymes, constitutes a considerable part of the total cost for the process of producing products from lignocellulosic material, it would be desirable to improve the efficiency of the enzymatic hydrolysis of lignocellulosic materials, e.g. to obtain more sugars from a certain enzyme dosage and time period, or to obtain the same amount of sugars from a lower enzyme dosage for the same time period. It is also desirable to achieve a certain amount of sugars with a certain enzyme dosage in a shorter time period, since this increases the production capacity and thereby allows for improved production and/or decreased costs of investment. Improving the efficiency of the enzymatic hydrolysis of cellulose may significantly contribute to commercialization of products based on lignocellulose-derived sugars.

Furthermore, the inhibitory effects of the by-products formed during the pretreatment may result in a less effective fermentation process due to the negative effects on the microorganisms.

### SUMMARY

In view of the above, it is an object of the present invention to provide improvements with respect to methods and systems for treating lignocellulosic biomass, particularly to reduce inhibitory substances of a lignocellulosic biomass-based material and to alleviate the negative effects of the inhibitory substances on downstream processes of the pretreatment.

According to a first aspect of the present invention, a method for reducing inhibitory substances of a lignocellulosic biomass-based material is provided. The method comprises:
- pretreating a lignocellulosic biomass material in a pretreatment arrangement, the pretreating including subjecting the lignocellulosic biomass material to an elevated temperature and pressure in a reactor vessel of the pretreatment arrangement to provide a pretreated biomass material comprising inhibitory substances;
- hydrolysing the pretreated biomass material in a hydrolysis unit with at least one aqueous hydrolysing liquid comprising saccharification enzymes, under conditions in which at least a part of the pretreated biomass material is hydrolysed to a hydrolysate, said hydrolysate comprising fermentable sugars;
- adding at least one reducing agent selected from sulfur oxyanions and sulfhydryl reagents to the lignocellulosic biomass-based material downstream of the reactor vessel for reducing inhibitory substances related to saccharification enzymes and/or fermenting microorganism, wherein the concentration of the reducing agent is adapted based on the temperature of the material where it is added, and/or wherein the reducing agent is added to the material having a temperature of T °C, T °C being more than 50 °C.

The invention is generally based on the finding that the addition of a reducing agent to improve treatment of the lignocellulosic biomass-based material downstream of the reactor vessel can be adapted based on the temperature of the point of addition of the reducing agent. Hereby, a more precise amount of reducing agent considering the characteristics of the pretreated biomass material can be achieved. The inventors have found that the amount of reducing agent may be reduced with increasing temperatures of the point of addition. For example, the amount of reducing agent may be reduced by adding the reducing agent to the lignocellulosic biomass-based material downstream of the reactor vessel (e.g. to the pretreatment slurry or the pretreated biomass material) at a point of addition where the lignocellulosic biomass-based material has an elevated temperature (i.e. a temperature at least higher than room temperature, or higher than 25 °C, further specified below). Moreover, a moderate increase of the temperature of the lignocellulosic biomass-based material at the point of addition of the reducing agent is related to a relatively higher amount of reducing agent compared to a significant increase of the temperature. Also, in case the temperature of the lignocellulosic biomass-based material at the point of addition of the reducing agent for some reason vary, the concentration of the reducing agent may be correspondingly adapted. The point of addition of the reducing agent may be referred to as the point of application of the reducing agent. The concentration of the reducing agent is typically referring to the final concentration after addition to the lignocellulosic biomass-based material at the point of addition of the reducing agent.

Thus, the method of the improve the efficiency in the process of producing sugars and downstream products from a lignocellulosic biomass material, with an adequate amount of reducing agent, e.g. a reduced amount of reducing agent. Hereby, the efficiency of e.g. the enzymatic hydrolysis in saccharification of lignocellulosic based materials can be improved, or at least maintained with a less amount of reducing agent. Additionality or alternatively, the efficiency of the fermentation of the sugars can be improved, or at least maintained with a less amount of reducing agent. Thus, the production capacity, or cost-effectiveness of the production, in saccharification and/or fermentation of lignocellulosic based materials may be improved.

It should be understood that the addition of reducing agent differs from the known addition of agents, such as sulfuric acid, sulfur dioxide and sulfite, to the pretreatment (e.g. to an impregnation vessel arranged upstream of the reactor vessel) to make pretreatment performed at high temperatures more efficient (i.e. the higher temperature in the reactor vessel). Addition of these agents in pretreatment utilizes their acidic properties and targets the lignocellulosic substrate and its susceptibility to enzymatic hydrolysis, while addition of reducing agents after the reactor vessel according to the present invention is contemplated to target at least enzyme inhibitors in the pretreatment liquid. According to at least one example embodiment, the method comprises pretreating the lignocellulosic biomass material in a pretreatment arrangement by subjecting the lignocellulosic biomass material to an impregnation of e.g. sulfuric acid, sulfur dioxide and/or sulfite in an impregnation vessel upstream of the reactor vessel, or in the reactor vessel prior to subjecting the lignocellulosic biomass material to the elevated temperature and pressure. Moreover, the inhibitory substances related to saccharification enzymes and/or fermenting microorganism target in the present invention is mainly originating from the lignocellulosic biomass material and the treatment of the same at an elevated temperature and pressure in the reactor vessel, and thus the reducing agent and the concentration thereof is typically adapted to target such inhibitory substances. The inhibitory substances related to saccharification enzymes and/or fermenting microorganism may be referred to as inhibitory substances for, or to, saccharification enzymes and/or fermenting microorganism, i.e. substances being inhibitory for the saccharification enzymes and/or fermenting microorganism.

According to at least one example embodiment, the lignocellulosic biomass-based material downstream of the reactor vessel subject to the addition of the reducing agent may be the lignocellulosic biomass-based material downstream of the pretreatment arrangement, i.e. the pretreated slurry or any fraction thereof, or the hydrolysate with the primary target to reduce inhibitory substances for fermenting microorganism. The lignocellulosic biomass-based material downstream of the reactor vessel subject to the addition of the reducing agent may alternatively be the direct discharge of lignocellulosic biomass-based material from the reactor vessel and/or the lignocellulosic biomass-based material in a potential steam-explosion step arranged downstream, or at the end of, the reactor vessel.

According to at least one example embodiment, the reducing agent is added to the lignocellulosic biomass material prior to hydrolysing the pretreated biomass material in a hydrolysis unit. Hereby, the addition of the reducing agent leads to improving enzymatic hydrolysis in saccharification of the lignocellulosic biomass material. Saccharification refers here to conversion or hydrolysis of lignocellulosic biomass material into e.g. mono- and disaccharides. The improvement may involve e.g. increasing the cellulose consumption rate, increasing the total amount of sugars produced during the pretreatment and the hydrolysis, increasing the sugar yield on used lignocellulose during pretreatment and hydrolysis, or increasing the volumetric primary target chemical productivity. It may also involve maintaining one or more of these parameters using a lower concentration of enzyme or a shorter time period in the hydrolysis step. In the context of the present disclosure, "sugars" refers to fermentable saccharides, or fermentable sugars, such as monosaccharides, disaccharides and oligosaccharides.

According to at least one example embodiment, the method of the invention is directed to treatment of lignocellulosic biomass materials, such as wood chips. The term lignocellulosic biomass material includes lignocellulose-derived material, i.e. material obtainable from lignocellulosic material, which comprises cellulose, lignin and possibly hemicellulose. The lignocellulose-derived material may for example be derived from wood materials or forestry residues, such as wood chips, sawmill or paper mill discards, or agricultural residues, e.g. corn stover or corn cobs. As an example, the lignocellulose-derived material may be wood-derived material or sugarcane bagass-derived material. Depending on the geographical location, wood or sugarcane bagass may be available in large quantities, making them attractive as raw materials.

Lignocellulose consists mainly of lignin and two classes of polysaccharides, cellulose and hemicellulose. In saccharification of lignocellulose, the polysaccharides are hydrolysed to sugars, including e.g. disaccharides and monosaccharides. As used herein, hydrolysis refers to subjecting the lignocellulosic biomass-based material to hydrolysing conditions such that free sugars become accessible in a hydrolysate for further treatment, e.g. fermentation. The free sugars are useful in the manufacture of desired products, such as alcohols, preferably ethanol, and organic acids.

Thus, according to the method of the invention, the lignocellulosic biomass material is subjected to a pretreatment, in which the hemicellulose is degraded and the cellulose is made increasingly accessible to cellulolytic enzymes or acidic hydrolysis. Pretreating a lignocellulosic biomass material refers to subjecting the lignocellulosic material to conditions such that the cellulose becomes more accessible during subsequent hydrolysis. The pretreatment may involve one or several pretreatment methods known to the skilled man. As an already mentioned, the pretreatment may be performed at elevated temperature and pressure with or without impregnation, typically using a dilute mineral acid, such as sulfuric acid, or an alkali. The pretreatment may thus involve impregnation, which refers to impregnating of the cellulosic material with an impregnation fluid, followed by heating. In the case of acid pretreatment, the impregnation fluid may be an acid solution, such as a mineral acid solution. The impregnation may also be performed with a gas, such as a SO2 gas or CO2 gas, or with the combination of a gas with a liquid to obtain e.g. sulfurous acid or carbonic acid. The impregnation may also be performed with sulfuric acid. The elevated temperature may be achieved by steaming, a process used to drive air out from the cellulosic biomass to facilitate hydrolysis of the cellulose. Steaming is a well-known method for pretreating e.g. lignocellulosic biomass material. As another example, the pretreatment may involve steam explosion, a process that combines steam, rapid pressure releases and hydrolysis for rupturing cellulosic fibers. Depending on the type of pretreatment, it may be desirable to neutralize the pretreated lignocellulosic material prior to the subsequent enzymatic hydrolysis. For example, the pretreated biomass material may be neutralized by means of a buffer or an addition of NaOH or ammonia. Also, CaOH2 may be used.

According to at least one example embodiment, the pretreating involves subjecting the lignocellulosic biomass material to an elevated temperature in the range of 120 - 220 °C, and an elevated pressure of 10 - 25 bar, typically for 1-60 min. Additionally, the pretreating comprises subjecting the lignocellulosic biomass material to sulfuric acid, sulfurous acid or sulfur dioxide, at, or prior to, the elevated temperature.

The pretreatment of the lignocellulosic biomass material typically provides a slurry of pretreated lignocellulosic material, or a pretreated slurry, wherein the hemicellulose is degraded and the cellulose is made increasingly accessible to cellulolytic enzymes or acidic hydrolysis. The suspended solids content of the slurry is typically in the range of from 5 to 40% (w/v), such as from 8 to 30% (w/v), such as from 12 to 20% (w/v).

According to at least one example embodiment, the method further comprising separating the pretreated biomass material into a liquid fraction and a solid fraction, wherein the at least one reducing agent is added to the liquid fraction and/or the solid fraction or a suspension thereof.

In other words, the pretreated slurry may be subject to separation in which the slurry is separated into a liquid fraction and a solid fraction. Typically, the solid fraction is washed and diluted to provide a suspension used for the enzymatic hydrolysis in the absence of the liquid fraction. The liquid fraction can be separated from the solid fraction of the pretreated slurry in various ways that are known to the skilled person, e.g. by allowing the solids to settle and decanting the liquid fraction, by centrifugation, by filtration, or combinations of these methods. The suspended solids content of the liquid fraction is typically below 1.0% (w/v), such as below 0.5% (w/v), such as below 0.1% (w/v). According to at least one example embodiment, at least one of the liquid fraction and solid fraction, or any derivates thereof (e.g. the solid fraction subsequent to be subject to washing and dilution) is subject to the addition of the at least one reducing agent. It is noted that while cellulases in general act on solid phase material, cellobiases are active in the liquid phase. The solid fraction and/ or the liquid fraction may then be subjected to enzymatic hydrolysis by cellulases or cellobiases in the presence of the at least one reducing agent. However, it is for practical purposes and for overall process efficiency and economy desirable to utilize the whole slurry of pretreated lignocellulosic biomass material in the subsequent enzymatic hydrolysis process, which is further exemplified below.

Following the pretreating, the lignocellulosic biomass-based material is subjected to hydrolysis or enzymatic hydrolysis. Enzymatic hydrolysis refers to a hydrolysis reaction catalysed by at least one enzyme. The at least one enzyme may be at least one saccharification enzyme, which refers to at least one enzyme that can convert or hydrolyse cellulosic biomass into fermentable saccharides, such as monosaccharides and/or disaccharides. Such saccharification enzymes may be glycosidases, which hydrolyse polysaccharides. Examples of glycosidases include cellulose-hydrolysing glycosidases, such as cellulases, endoglucan-ases, exoglucanases, cellobiohydrolases and β-glucosidases, hemicellulose hydrolyzing glycosidases, such as xylanases, endoxylanases, exoxylanases, β-xylosidases, arabinoxylanases, mannanases, galactanases, pectinases and glucuronases, , or any enzymes in the group of enzymes found in EC 3.2.1.x, such as EC 3.2.1.4, where EC is the Enzyme Commission number. In one embodiment, the at least one enzyme originates from filamentous fungi including Hypocrea jecorina (Trichoderma reseei).

Thus, for the embodiments in which the at least one reducing agent is added prior to the hydrolysis, the enzymatic hydrolysis inhibitory properties can be decreased by addition of at least one reducing agent downstream of the reactor vessel, and by the adaption of the concentration of the reducing agent to the temperature of application of the reducing agent, the decrease of the inhibitory properties can be efficiently achieved. The lignocellulosic biomass-based material with the decreased inhibitory properties are then subjected to enzymatic hydrolysis in the presence of the at least one reducing agent. As described before, this step provides a saccharified lignocellulosic biomass-based material, of the sugar-containing fermentation media with e.g. mono- di- and/or oligosaccharides, which can be fermented or otherwise utilized as a substrate for production of a primary target chemical.

According to at least one example embodiment, the lignocellulosic biomass material is discharged from the reactor vessel as a pretreatment slurry having an elevated temperature of at least 50 °C, and the at least one reducing agent is added to the pretreatment slurry at the elevated temperature.

Hereby, the residual heat from the pretreatment arrangement, and in particular from the reactor vessel, can be utilized. By adding the reducing agent to the pretreatment slurry (or pretreated slurry out of the pretreatment arrangement) at the elevated temperature of at least 50 °C, the concentration of the reducing agent can be kept relatively low, at least compared to a scenario in which the reducing agent is added to the lignocellulosic biomass material at a temperature close to room temperature. The pretreated slurry discharged from the pretreatment arrangement is then subjected to enzymatic hydrolysis in the presence of the at least one reducing agent.

According to at least one example embodiment, the at least one reducing agent is added to the pretreated slurry prior any separation procedure of the pretreated slurry. According to at least one example embodiment, the pretreated slurry is not subjected to a separation procedure. For practical purposes, it is convenient to avoid separation procedures and separate treatments for different fractions of the pretreated biomass material. Regardless of if the pretreated slurry is subjected to a separation procedure or not, without being bound by any theory, it is believed that the addition of the reducing agent according to the invention improves the treatment of the solids in the lignocellulosic biomass-based material, and in particular the sulfonation of the solids, and thus improves the downstream process of hydrolysis and/or fermentation.

According to at least one example embodiment, the at least one reducing agent is added to the pretreatment slurry at, or just after, the outlet of the reactor vessel. According to at least one example embodiment, the at least one reducing agent is added to the pretreated slurry at, or just after, the outlet of the pretreatment arrangement. According to at least one example embodiment, the at least one reducing agent is added to the pretreatment slurry or the pretreated slurry. That is, the at least one reducing agent may be added in the pretreatment arrangement, or at, or just after, the outlet of the pretreatment arrangement. If the outlet of the reactor vessel equals the outlet of the pretreatment arrangement, the pretreatment slurry and the pretreatment slurry is referring to the same lignocellulosic biomass-based material. According to at least one example embodiment, the method comprises subjecting the lignocellulosic biomass material to a steam explosion step at the end of, or downstream of, the reactor vessel. According to at least one example embodiment, the at least one reducing agent is added to the lignocellulosic biomass material in, or just after, the steam explosion step. The steam explosion step may e.g. be performed in a steam explosion configuration arranged in the pretreatment arrangement, at the end of, or downstream, the reactor vessel. The pretreated slurry out of the pretreatment arrangement is then subjected to enzymatic hydrolysis in the presence of the at least one reducing agent, in which the reducing agent has reduced the inhibitory substances for the saccharification enzymes.

According to at least one example embodiment, in which the concentration of the reducing agent is adapted based on the temperature of the material where it is added, and wherein the concentration of the reducing agent is between 5 and 50 mM and the temperature of the material is between 25 °C and 140 °C, or wherein the concentration of the reducing agent is between 5 and 40 mM and the temperature of the material is between 40 °C and 140 °C, or wherein the concentration of the reducing agent is between 5 and 30 mM and the temperature of the material is between 50 °C and 140 °C.

Thus, for higher temperatures of the lignocellulosic biomass-based material, a more narrow range with a lower top value of the concentration of the reducing agent may be used with the same result in reduction of inhibitory substances for saccharification enzymes and/or fermenting microorganism. According to at least one example embodiment, the concentration of the reducing agent is between 5 and 30 mM and the temperature of the material is between 75 °C, or 80 °C, or 85 °C, or 95 °C, or 110 °C and 125 °C or 130 °C or 135 °C or 140 °C.

According to at least one example embodiment, in which the concentration of the reducing agent is adapted based on the temperature of the material where it is added, and wherein the concentration of the reducing agent:
is between 12.5 to 50 mM in the temperature range of the material between 25 °C to 50 °C, and between 8.5 to 25 mM in the temperature range of the material between 50 °C and 75 °C, and between 5 to 15 mM in the temperature range between 75 °C and 140 °C.

According to at least one example embodiment, in which the concentration of the reducing agent is adapted based on the temperature t of the material where it is added, and wherein the concentration C(t) of the reducing agent is determined based on the equation C(t)=-k^{∗}t + (Const+/- 30%), wherein k and Const are positive numbers.

Hereby, a simply yet effective way of adapting the concentration based on the temperature of the material where it is added, is provided. For the equation C(t)=-k^{∗}t + (Const+/- 30%), C(t) is the temperature dependent added concentration of the reducing agent, and t is the temperature in Celsius (°C) of the material where the reducing agent with concentration C(t) is added. The temperature t °C may be the same as the temperature T °C (T °C being at least 50 °). The positive number of k is e.g. 0.1 and 1, such as e.g. between 0.2 and 0.6, and the positive number of Const is e.g. 40. The term Const+/-30 % should be interpreted as a variation of the value of the constant with + 30 % and - 30 %, i.e. from 28 to 52.

According to at least one example embodiment, in which the reducing agent is added to the material having a temperature of T °C, and T °C is at least 50 °C, or at least 75 °C, or at least 80 °C, or at least 95 °C, or at least 110 °C, and at most 125 °C, or at most 130 °C, or at most 135 °C, or at most 140 °C.

Correspondingly to the embodiment in which the at least one reducing agent is added to the pretreatment slurry, or pretreated slurry, at the elevated temperature, the elevated temperature being at least 50 °C, or at least 75 °C, or at least 80 °C, or at least 95 °C, or at least 110 °C, and at most 140 °C.

According to at least one example embodiment, the at least one reducing agent is configured to reduce inhibitor substances both related to enzymes and microorganisms, i.e. both saccharification enzymes and fermenting microorganism. Hereby, the addition of the reducing agent improves both hydrolysis and fermentation.

According to at least one example embodiment, the method comprises subjecting the hydrolysate with the fermentable sugars to fermentation in an aqueous liquid utilizing at least one fermenting microorganism under conditions in which at least a part of the fermentable sugars is fermented into a primary target chemical.

Thus, the fermentation of the pretreated and enzymatically hydrolysed lignocellulosic biomass material may be performed by a fermenting microorganism, which refers to an organism that is capable of fermenting sugars, e.g. mono- or disaccharides into a fermentation product, e.g. a primary target chemical of the method. The fermenting microorganism may be at least one eukaryotic or prokaryotic microorganism, such as bacteria and/or yeast. Examples of bacteria and yeasts which are capable of fermenting saccharides into other chemical compounds are known to the skilled person. Yeasts from Saccharomyces, Pichia and Candida may be used as the fermenting organism. The fermenting organism may for example be wild type, mutant or recombinant Saccharomyces cerevisiae (S. cerevisiae). Using S. cerevisiae for producing a fermentation product is advantageous since S. cerevisiae is well established with regard to industrial fermentation and provides for a high product yield.

According to at least one example embodiment, the hydrolysis is performed separately from the fermentation, or the hydrolysis and the fermentation are performed simultaneously in a single step.

According to at least one example embodiment, the fermentation is a simultaneous saccharification and fermentation (SSF) of a pretreated biomass material. An SSF process refers to a process in which enzymatic hydrolysis and fermentation is performed simultaneously in a fermenter. Thus, in an SSF process, fermentable saccharides are prepared directly in a fermenter by enzymatic hydrolysis of the pretreated biomass material, and the resulting saccharides are converted into a fermentation product, such as a primary target chemical. Further, the fermentation may be a consolidated bioprocess (CBP), in which the biocatalyst that convert the monosaccharides also produces the enzymes that hydrolyse the pretreated biomass material.

According to at least one example embodiment, the hydrolysate that is subjected to fermentation is obtained from enzymatic hydrolysis of the pretreated biomass material in a step separate from the fermentation step. Consequently, the enzymatic hydrolysis and the fermentation may be performed as two separate process steps (separate hydrolysis and fermentation, SHF). This may e.g. be advantageous if the fermentation reaction and the enzymatic reaction have different optimal temperatures. As an example, the temperature during enzymatic hydrolysis may be kept higher than the temperature during fermentation, thus facilitating the use of thermophilic enzymes. While the enzymatic hydrolysis is generally performed prior to the fermentation step, it is noted that the fermented material or parts thereof, e.g. a liquid fraction, may be returned to the to the original slurry and facilitate liberation of monosaccharides from the solids in the slurry, which may then be subjected to fermentation.

According to at least one example embodiment, the at least one reducing agent is added to the lignocellulosic biomass-based material downstream of the reactor vessel, but upstream of the fermentation (e.g. upstream of an SSF unit or a separate fermentation unit). According to at least one example embodiment, the at least one reducing agent is added to the lignocellulosic biomass-based material downstream of the hydrolysis, but upstream of the fermentation (e.g. in the SSF unit or between the hydrolysis unit and the fermentation unit in a SHF process). For example, by dewatering the hydrolysate and increasing the concentration of the fermentable sugars, an increase of any inhibitory substances present in the hydrolysate is to be expected, why the addition of the reducing agent to such hydrolysate may be advantageous. Thus, the present approach may also include reducing agents to achieve improved fermentability of lignocellulose hydrolysates, the goal in the latter case being to alleviate the effect of inhibitors on the fermenting microorganism as an alternative to, or in addition to, the enzymatic hydrolysis. Preferably, the at least one reducing agent is added to the lignocellulosic biomass-based material downstream of the reactor vessel where the temperature is the highest.

The fermentation inhibition by the inhibitory substances typically has a negative effect on the fermentation reaction, e.g. decreasing of the rate of the fermentation reaction or the total amount of target product produced in the fermentation reaction (e.g. as a result of a slower conversation of sugar, e.g. glucose, into the target product, e.g. ethanol). By the addition of the reducing agent, the fermentation inhibition due to the inhibitory substances can be decreased, and negative effects reduced or omitted. Consequently, decreasing the fermentation inhibition may be detoxification or conditioning of a material subjected to fermentation, i.e. decreasing the effect of one or more properties of the material subjected to fermentation, which properties are inhibiting the fermenting organism's conversion of a substrate to the target chemical. For example, decreasing the fermentation inhibition may be increasing the saccharide consumption rate, such as the glucose consumption rate, increasing the total amount of target chemical produced during fermentation, increasing the target chemical yield on consumed saccharide during fermentation, i.e. increasing the number of target chemical molecules produced by each consumed saccharide molecule, or increasing the volumetric target chemical productivity. By the addition of the reducing agent according to the invention, an improvement in fermentability can be achieved with a relatively small addition of reducing agent and further, the reducing agent is compatible with enzymes and fermenting organisms such as yeast, thus resulting in marginal or no influence on enzyme or yeast performance.

According to at least one example embodiment, the inhibitory substance targeted by the reducing agent of the present invention is formaldehyde.

According to at least one example embodiment, the inhibitory substance(s) targeted by the reducing agent of the present invention is derivable from at least one of the following: lignin degradation products such as phenolic aldehydes (e.g. coniferyl aldehyde) or phenolic acids (e.g. ferulic acid), sugar degradation products, such as furan aldehydes (e.g. furfural and HMF), quinones, such as benzoquinone. The targeted inhibitory substance(s) may be naturally occurring substances from the raw biomass material such as various types of extractives, e.g. phenolic substances such as tannins and flavonoids.

Thus, in summary, the lignocellulosic biomass material may be pretreated to form a pretreated slurry, hydrolyzed to form a hydrolysate, and fermented to produce a fermentation product, at least being a primary target chemical of the process. All of the lignocellulosic biomass-based materials, from downstream of the reactor vessel in the pretreatment arrangement, to the pretreated slurry, the hydrolysate and up to the fermentation, may be subject to the addition of the at least one reducing agent wherein the concentration of the reducing agent is adapted based on the temperature of the lignocellulosic biomass-based material where it is added, and/or wherein the reducing agent is added to the lignocellulosic biomass-based material having a temperature of T °C, T °C being more than 50 °C (with embodiments as described earlier).

According to at least one example embodiment, the primary target chemical is a fermentation product selected from the group consisting of alcohol, organic acid or aliphatic acid.

For example, the fermentation product is ethanol, butanol, lactic acid, levulinic acid and/or succinic acid.

Thus, as set out above, the present method provides a saccharified lignocellulosic based material, e.g. mono- and disaccharides, which can be fermented or otherwise utilized as a substrate for production of desired target compounds. These sugars can be utilized as substrates in various chemical and biochemical (e.g. enzymatic) methods for production of desired target compounds. By way of example, the sugars can be used in a thermochemical process to produce levulinic acid, which in turn is an intermediate in the synthesis of polymers, plastics and pharmaceuticals, or be used for biodegradable plastics such as PLA by means of lactic acid. It is also a precursor in the industrial production of other chemical commodities such as methyltetrahydrofuran, valerolactone, and ethyl levulinate. According to one example embodiment, the method of the invention further involves the step of utilizing the pretreated and enzymatically hydrolyzed lignocellulosic biomass-based material as a substrate for production of target compounds. The present method also offers a way to achieve more efficient saccharification of lignocellulose in the manufacture of fermentation products from lignocellulose hydrolysates. Examples of fermentation products according to the invention include alcohols, acids, alkanes, alkenes, aromatics, aldehydes, ketones, biopolymers, proteins, peptides, amino acids, vitamins, antibiotics and other pharmaceuticals. In one embodiment, the fermentation product is selected from the group consisting of ethanol, butanol and succinic acid. A preferred fermentation product is ethanol. Consequently, the method according to the invention also provides for an efficient production of fuels, such as ethanol, and other chemicals from lignocellulosic biomass materials.

According to at least one example embodiment, the at least one reducing agent is sulfur oxyanions, or is a substance which when dissolved in liquid provides sulfur oxyanions. Sulfur oxyanions are capable of reducing inhibitor substances both related to enzymes and microorganisms, i.e. both saccharification enzymes and fermenting microorganism. Typically, a lignocellulosic biomass-based material treated with sulfur oxyanions results in substances with a negative charge, tending to reduce the inhibitory substances.

In general, a "reducing agent" refers to a chemical agent capable of causing the reduction of another substance as it itself is oxidized, i.e. a chemical agent capable of donating an electron in an oxidation-reduction reaction. The reducing agent is compatible with fermenting microorganisms such as yeast.

According to at least one example embodiment, the at least one reducing agent is selected from sulfite, dithionite and dithiothreitol.

These reducing agents have shown to be suitable for decreasing the inhibition of enzymatic hydrolysis. Dithionite and sulfite (hydrogen sulfite at the pH used for enzymatic saccharification) are sulfur oxyanions. Sulfite (SO₃²⁻), derived from sulfur dioxide gas or from addition of salt, is used in several large-scale industrial processes, including pretreatment of lignocellulosic substrates. Dithionite (S₂O₄²⁻) is an industrial chemical used in the pulp and paper industry for reductive bleaching and in the textile industry as a reducing agent in dyeing processes. Hence, both sulfite and dithionite are available in large quantities. Further, it is to be understood the reducing agent may comprise sulfite and/or dithionite in salt form, i.e. complexed with different cations. Examples include Na₂SO₃, NaHSO₃, KHSO₃, and Na2S2O4. Dithionite, when dissolved in the lignocellulosic biomass-based material, will form sulfite ion, and thus have a corresponding effect as adding sulfite. Correspondingly, a salt comprising sulfite or dithionite will, after being dissolved in the lignocellulosic biomass-based material, form sulfite ions (either directly, or via the previously described dissolvement of dithionite). Dithiothreitol (DTT), also known as Cleland's reagent, represents sulfhydryl compounds. DTT is interesting in this context considering that it is known to efficiently reduce disulfide bonds in proteins. Reducing agents like DTT could therefore tentatively have a detrimental effect by destabilizing proteins with disulfide bridges.

According to at least one example embodiment, the at least one reducing agent is selected from sulfite and dithionite. According to at least one example embodiment, the at least one reducing agent is sulfite. According to at least one example embodiment, the at least one reducing agent is dithionite. According to at least one example embodiment, the at least one reducing agent is sulfite and/or dithionite.

For example, the reducing agent is dithionite, and the dithionite may be added in an amount such that the concentration of dithionite is above 5 mM, or in the range of 5-30 mM, such as 5-25 mM, such as 5-20 mM. For example, the concentration of dithionite is in the range of 5-20 mM or in the range of 5-12.5 mM. For example, the concentration of dithionite is above 12.5 mM or between 12.5 mM and 20 mM or 30 mM or 40 mM or 50 mM in the temperature range of the material between 25 °C to 50 °C, and/or above 8.7 mM or between 8.7 and 12.5 or 20 mM or 25 mM in the temperature range of the material between 50 °C and 75 °C, and/or above 5 mM or between 5 and 8.7 mM or 12.5 mM or 15 mM in the temperature range between 75 °C and 140 °C. For example, the reducing agent is sulfite, and the sulfite is added in an amount such that the concentration of sulfite is above 5 mM, or in the range of 5-50 mM, such as 5-40 mM, such as 5-30 mM. As yet another example, the reducing agent is dithiothreitol, and the dithiothreitol is added in an amount such that the concentration of dithiothreitol is above 5 mM, or in the range of 5-30 mM, such as 5-25 mM, such as 5-20 mM.

These concentrations of dithionite, sulfite and dithiothreitol, respectively, have shown to be suitable for decreasing inhibitory substances, and as described earlier in the application text, preferably added in an amount in the lower span of the ranges as a result of the relatively higher temperature. It may however be disadvantageous for a subsequent fermentation process to add more than 100 mM of sulfite, dithionite or dithiothreitol.

Sulfur oxyanions, such as sulfite and dithionite, can also efficiently improve the fermentability of lignocellulose hydrolysates to levels that give similar yields of fermentation product as are obtained with reference fermentations based on synthetic sugar solutions. The usefulness of sulfur oxyanions for improving pretreatment and fermentability makes the approach to enhance the efficiency of enzymatic hydrolysis presented herein even more attractive from an industrial perspective, since the same chemical can be used for different purposes in different parts of the process.

According to a second aspect of the invention, there is provided a novel use of at least one reducing agent for decreasing the enzymatic hydrolysis inhibitory properties, and/or decreasing the microorganisms fermentation inhibitory properties of a pretreated lignocellulosic biomass-based material by adapting the concentration of the reducing agent based on the temperature of the material where it is added, and/or by adding the reducing agent to the material having a temperature of T °C, T °C being more than 50 °C.

Effects and features of the second aspect of the invention are largely analogous to those described above in connection with the first aspect of the invention. Embodiments mentioned in relation to the first aspect of the invention are largely compatible with the second aspect of the invention, of which some are exemplified below.

According to at least one example embodiment, the at least one reducing agent is selected from sulfur oxyanions and sulfhydryl reagents, e.g. the at least one reducing agent is selected from sulfite, dithionite and dithiothreitol.

According to a third aspect of the invention, a system for reducing inhibitory substances of a lignocellulosic biomass-based material is provided. The system comprises:
- a pretreatment arrangement for pretreating a lignocellulosic biomass material, the pretreatment arrangement comprises a reactor vessel configured to subject the lignocellulosic biomass material to an elevated temperature and pressure to provide a pretreated biomass material comprising inhibitory substances;
- a hydrolysis unit configured to treat the pretreated biomass material with at least one aqueous hydrolysing liquid comprising saccharification enzymes under conditions in which at least a part of the pretreated biomass material is hydrolysed to a hydrolysate comprising fermentable sugars;
- a feeding device configured to add at least one reducing agent selected from sulfur oxyanions and sulfhydryl reagents to the lignocellulosic biomass-based material downstream of the reactor vessel for reducing inhibitory substances related to saccharification enzymes and/or fermenting microorganism, wherein the feeding device is configured to adapt the concentration of the reducing agent based on the temperature of the material where it is added, and/or wherein the feeding device is configured to add the reducing agent to the material having a temperature of T °C, T °C being more than 50 °C.

Effects and features of the third aspect of the invention are largely analogous to those described above in connection with the first aspect of the invention. Embodiments mentioned in relation to the first aspect of the invention are largely compatible with the third aspect of the invention, of which some are exemplified below. Particularly embodiments related to the adaption of the concentration of the reducing agent based on the temperature of the material where it is added, and embodiments related to the temperature of T °C, are applicable to the third aspect of the invention.

According to at least one example embodiment, the system comprises means for subjecting the hydrolysate with the fermentable sugars to fermentation in an aqueous liquid utilizing at least one fermenting microorganism under conditions in which at least a part of the fermentable sugars is fermented into a primary target chemical.

For example, the means for subjecting the hydrolysate with the fermentable sugars to fermentation in an aqueous liquid is a fermentation unit or a combined SSF unit for a simultaneous saccharification and fermentation process.

According to at least one example embodiment, the system further comprises a measuring device configured to measure the temperature of the lignocellulosic biomass-based material downstream of the reactor vessel, and at the point of addition of the at least one reducing agent.

Hereby, the concentration of the reducing agent can be easily adapted based on the prevailing conditions, in particular the prevailing temperature, of the lignocellulosic biomass-based material at the point of addition or application of the reducing agent. For example, the system comprises a control unit configured to use the temperature measured by the measuring device, or otherwise determined, wherein the control unit is further configured to adjust the concentration of the at least one reducing agent added to the lignocellulosic biomass-based material in response to the temperature. The temperature may be measured in the vicinity of the point of addition of the at least one reducing agent, and the measured temperature may be used to estimate the temperature at the point of addition.

According to at least one example embodiment, the pretreatment arrangement further comprises a steam explosion configuration arranged downstream, or in an outlet region of, the reactor vessel.

For example, the feeding device comprises a pipe for transporting the reducing agent, and wherein the pipe comprises an outlet arranged and configured to supply the reducing agent to the lignocellulosic biomass-based material downstream of the reactor vessel. The outlet of the pipe may e.g. be arranged at, or just after, the outlet of the reactor vessel or in, or just after, the steam explosion configuration. According to at least one example embodiment, the outlet of the pipe is arranged between the reactor vessel and the hydrolysis unit.

For example, the system may comprise additional units and components known to those skilled in the art. For example, a separation unit may be arranged between the reactor vessel or pretreatment arrangement and the hydrolysis unit, and/or between the hydrolysis unit and the fermentation unit.

### BRIEF DESCRIPTION OF THE DRAWINGS

The various aspects of the present invention, including its particular features and advantages, will be readily understood from the following detailed description and the accompanying drawings, in which:
Fig. 1 schematically illustrates a system for treatment of lignocellulosic biomass material including control for reducing inhibitory substances in accordance with example embodiments of the invention,
Fig. 2 schematically illustrates a system for treatment of lignocellulosic biomass material including control for reducing inhibitory substances in accordance with yet another example embodiments of the invention,
Fig. 3 schematically illustrates the steps of a method for reducing inhibitory substances of a lignocellulosic biomass-based material in accordance with example embodiments of the invention, and
Fig. 4 is a graph showing the effects of addition of a reducing agent to the lignocellulosic biomass-based material at different temperatures.

### DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

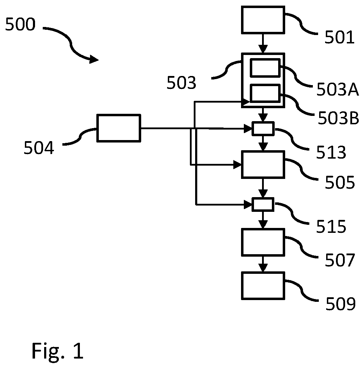

With reference to Fig. 1, the present disclosure provides a system 500 for treatment of lignocellulosic biomass comprising a supply unit 501 for supplying the lignocellulosic biomass material as a raw material (e.g. wood chips), a pretreatment arrangement 503 for pre-treating the lignocellulosic biomass material, the pretreatment arrangement 503 in Fig. 1 comprising an impregnation vessel 503A and a reactor vessel 503B wherein in the impregnation vessel 503A the lignocellulosic biomass material is impregnated with an additive facilitating degradation of the biomass material, and in the reactor vessel 503B the degradation is improved by exposing the biomass material to an elevated temperature and pressure, to provide a pretreated slurry of the pretreated lignocellulosic biomass material. The system further 500 comprises a hydrolysis unit 505 in which the pretreated slurry is subject to enzymatic hydrolysis by means of saccharification enzymes under conditions in which at least a part of the pretreated slurry is hydrolysed to a hydrolysate comprising fermentable sugars, the hydrolysis unit 505 being arranged downstream of and in fluid communication with the pretreatment arrangement 503. The system 500 further comprises a fermentation unit 507, such as a fermentation vessel 507, arranged downstream of and in fluid communication with the hydrolysis unit 505. In the fermentation vessel 507, the hydrolysate, or fermentation media, comprising fermentable sugars is fermented into a target chemical, e.g. ethanol, by means of a yeast. The system 500 may also comprise a product recovery unit 509, such as distillation or ion exchange chromatography, arranged downstream of and in fluid communication with the fermentation vessel 507.

During at least the pretreatment of the lignocellulosic biomass material, inhibitory substances having a negative impact on saccharification enzymes and/or fermenting microorganism are produced, mainly in the reactor vessel 503B due to the prevailing high temperature and pressure. To target such inhibitory substances, the system 500 is configured to reduce such inhibitory substances by a feeding device 504 configured to add at least one reducing agent to the lignocellulosic biomass-based material downstream of the reactor vessel 503 B. The reducing agent is typically selected from sulfur oxyanions and sulfhydryl reagents and thus reduce inhibitory substances for saccharification enzymes and/or fermenting microorganism.

In Fig. 1, four potential points of addition, or points of application, of the reducing agent by the feeding device 504 is shown, of which only one is needed. A first point of addition is arranged downstream of reactor vessel 503B, at the outlet of the pretreatment arrangement 503 (e.g. at, or just after a steam explosion configuration arranged at the end of the pretreatment arrangement 503). As this point of addition is within the pretreatment arrangement, the lignocellulosic biomass-based material to which the reducing agent is added, is referred to as a pretreatment slurry. A second point of addition is arranged downstream of the pretreatment arrangement 503 and upstream of the hydrolysis unit 505, i.e. addition of the reducing agent to the pretreated slurry. A third point of addition is arranged in the hydrolysis unit 50 and a fourth point of addition is arranged downstream of the hydrolysis unit 505 and upstream of the fermentation unit 507. For the second and fourth points of application, the feeding device 504 comprises a respective mixing unit 513, 515, the mixing units 513, 515 may for example be a simply T-connection or otherwise provided means for mixing the reducing agent into the lignocellulosic biomass-based material.

The feeding device 504, e.g. by means of a control unit, is configured to adapt the concentration of the reducing agent, at either one of the points of applications, based on the temperature of the material (i.e. the lignocellulosic biomass-based material) where it is added. Alternatively, the feeding device is configured to add the reducing agent to the material (i.e. the lignocellulosic biomass-based material) having a temperature of T °C, T being more than 50.

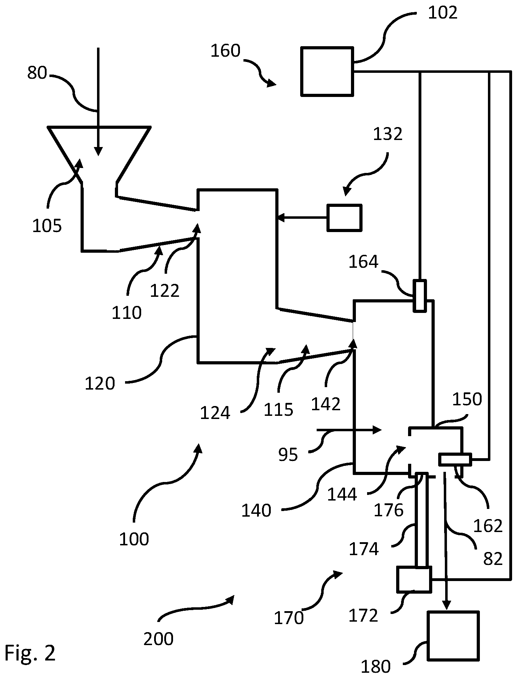

An example of such feeding device and a specific point of addition will now be described with reference to Fig. 2.

Fig. 2 illustrates an example system 200 comprising pretreatment arrangement 100 and a hydrolysis unit 180. The pretreatment arrangement is configured for pretreatment of lignocellulosic biomass material, and comprises an inlet hopper 105 configured to receive lignocellulosic biomass material 80 as a raw material, an impregnation vessel 120 arranged downstream of the inlet hopper 105, and a reactor vessel 140 arranged downstream of the impregnation vessel 120. The impregnation vessel 120 comprises an upstream inlet 122 for receiving the lignocellulosic biomass material 80 from the inlet hopper 105 as a lignocellulosic biomass material feed, and a downstream outlet 124 for discharging impregnated biomass to the reactor vessel 140. Correspondingly, the reactor vessel 140 comprises an upstream inlet 142 for receiving impregnated biomass from the outlet 124 of the impregnation vessel 120, and a downstream outlet 144 for discharging the lignocellulosic biomass-based material as a pretreatment slurry, and eventually out of the pretreatment arrangement 100 as a pretreated slurry 82. In an outlet portion of the pretreatment arrangement 100, at (or just after) an outlet 144 of the reactor vessel 140, a steam explosion configuration 150 is arranged. Subsequent to the pretreatment arrangement 100, the hydrolysis unit 180 configured to treat the pretreated biomass material 82 with at least one aqueous hydrolysing liquid comprising saccharification enzymes under conditions in which at least a part of the pretreated biomass material is hydrolysed to a hydrolysate comprising fermentable sugars, is arranged.

The pretreatment arrangement 100 further comprises an impregnation arrangement 132 configured to supply an impregnation substance, such as e.g. sulfuric acid, sulfur dioxide and/or sulfite to the lignocellulosic biomass in the impregnation vessel 120. The impregnation arrangement 132 may be configured to supply the impregnation substance to the impregnation vessel 120 in gaseous form, or as a liquid, and may comprise valves and other suitable equipment known to those skilled in the art.

The reactor vessel 140 may according to one example embodiment be a vertical reactor vessel. However, horizontal, as well as inclined, reactor vessels are also conceivable for the purpose of the present disclosure. As illustrated in Fig. 2, the reactor vessel 140 is a vertical reactor vessel comprising an upper portion comprising the inlet 142, and a lower portion comprising the outlet 144. The biomass thus flows from the inlet 142 to the outlet 144 by means of gravity, and no additional means to increase the flow of biomass within the reactor vessel 140 is required.

Thus, in more detail, the lignocellulosic biomass material 80 enters the pretreatment arrangement 100 via the inlet hopper 105, and is fed into the impregnation vessel 120 by means of a first plug screw feeder 110. In the impregnation vessel 120, the lignocellulosic biomass is treated or impregnated with the impregnation substance (e.g. SO2) by the use of the impregnation arrangement 132. Subsequently, the impregnated biomass material from the impregnation vessel 120 is fed to the reactor vessel 140 by means of a second plug screw feeder 115. The first and second plug screw feeders 110, 115 secures an even flow of biomass material into the impregnation vessel 120 and reactor vessel 140, respectively. Moreover, the first and second plug screw feeders 110, 115 may act as moisture adjusting units, here by compression which removes moisture from the biomass (dewatering). The pretreatment arrangement 100 is not limited to a specific type of inlet or feeding means, but any inlet or means for feeding biomass, known to those skilled in the art, may be used.

The lignocellulosic biomass material may be, but is not limited to, hardwoods, softwoods, sugarcane bagasse, energy cane, corn stover, corn cobs, corn fibers, straw from rice, wheat, rye and other crop or forestry residues. As illustrated by the arrow 95, steam and/or additional catalysts may in embodiments be added to the reactor vessel 140 for certain pretreatment conditions.

In the reactor vessel 140, and during operation, the impregnated biomass material from the impregnation vessel 120 is subject to an elevated temperature and pressure forming a biomass slurry. For example, the temperature in the reactor vessel 140 during pretreatment may be in the interval of 185 to 225°C, such as 200 to 215 °C. For example, the pressure in the reactor vessel 140 during pretreatment may be in the interval of 10 to 25 bar, such as 15 to 20 bar. Typically, the reactor vessel 140 has a circular or oval cross-section. The reactor vessel 140 may e.g. be cylindrical. In preferred embodiments, the reactor vessel 140 has a rotational symmetry with respect to a longitudinal center line.

During degradation of the lignocellulosic biomass material in the pretreatment arrangement 100, and in particular when the lignocellulosic biomass material is treated by the elevated temperature and pressure in the reactor vessel 140, inhibitory substances of saccharification enzymes and/or fermenting microorganism are formed. To handle such inhibitory substances, a reducing agent may be added to the lignocellulosic biomass-based material as previously described. The reducing agent is in Fig. 2 supplied to the pretreatment arrangement 100 downstream of the reactor vessel 140 by means of a feeding device 170.

The feeding device 170 comprises a supply unit 172 comprising the reducing agent (schematically illustrated) and a feeding pipe 174 having an outlet 176. In Fig. 2, the feeding device 170 is arranged and configured to supply the reducing agent from the supply unit 172, via the feeding pipe 174 and through the outlet 176 into the steam explosion configuration 150. Thus, the feeding device 170 is arranged and configured to supply the reducing agent to the pretreatment slurry within the pretreatment arrangement 100, but downstream of the outlet 144 of the reactor vessel 140. Alternatively, the outlet 176 may be arranged directly to a pipe or the like providing the pretreated slurry 82 out from the pretreatment arrangement 100 to the hydrolysis unit 180. Thus, the system 200 of Fig. 2 is configured to reduce inhibitory substances for the lignocellulosic biomass-based material.

Moreover, the pretreatment arrangement 100 in Fig. 2 comprises a measuring device 160 configured to measure the temperature of the lignocellulosic biomass-based material downstream of the reactor vessel 140 via a first measuring probe 162 arranged at the point of addition of the at least one reducing agent by the feeding device 170, i.e. in Fig. 2 in the steam explosion configuration 150. Alternatively, the first measuring probe 162 may be arranged at the pretreated slurry 82 out from the pretreatment arrangement 100. According to at least one example embodiment, the temperature at the point of addition of the at least one reducing agent is determined by measuring the temperature elsewhere, and estimate the temperature loss of the lignocellulosic biomass-based material up to the point of addition of the reducing agent. In Fig. 2, this is exemplified by the measuring probe 164 arranged and configured to measure the temperature in the reactor vessel 140. By knowing the temperature loss between the reactor vessel 140 and the point of addition of the reducing agent, here at the outlet of the reactor vessel 140 or in the steam explosion configuration 150, the temperature at the point of addition the reducing agent can be estimated. As a further example embodiment, the temperature of the lignocellulosic biomass-based material at the point of addition the reducing agent can be estimated or determined by other means known to those skilled in the art.

By knowing, or estimating, the temperature of the lignocellulosic biomass-based material at, or in the vicinity of, the point of addition the reducing agent, the concentration of the reducing agent can be easily adapted based on this temperature. For example, the pretreatment arrangement 100 may comprises a control unit 102 configured to use the temperature measured by the measuring device 160 (the measuring device 160 may comprise, or use, the control unit 102 when determining the temperature using the measurement probes 162, 164), or otherwise determined, wherein the control unit 102 is further configured to adjust the concentration of the at least one reducing agent added to the lignocellulosic biomass-based material in response to the temperature, by communicating with the feeding device 170.

As already discussed, a higher temperature of the lignocellulosic biomass-based material at the point of addition of the reducing agent implies that a lower concentration of the reducing agent can be used while still reaching a satisfactory reduction of the inhibitory substances. It should be noted that the point of addition, or point of application, of the reducing agent shown in Fig. 2 is particularly advantageous as the residual heat stemming from the reactor vessel 140 may be utilized.

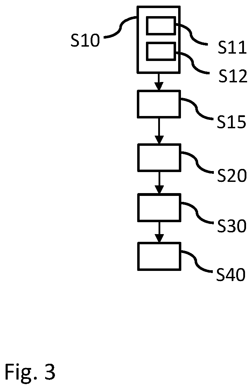

The invention will now be described with reference to the flow chart presented in Fig. 3. Fig. 3 schematically illustrates the steps of a method for reducing inhibitory substances of a lignocellulosic biomass-based material according to at least one example embodiment of the invention. The system, and any of its equipment, components and functions, may e.g. be those described with reference to Fig. 1 or Fig. 2.

First, the main steps of the method will be described, followed by a more detailed description including further optional steps. In step S10, a lignocellulosic biomass material is pretreated in a pretreatment arrangement, the pretreating S10 including subjecting S12 the lignocellulosic biomass material to an elevated temperature and pressure in a reactor vessel of the pretreatment arrangement to provide a pretreated biomass material comprising inhibitory substances. The step S10 may e.g. comprise a sub-step S11 of impregnating the lignocellulosic biomass material with an impregnation substance in an impregnation vessel, or in a portion of the reactor vessel, of the pretreatment arrangement. In step S20, the pretreated biomass material is hydrolysed in a hydrolysis unit with at least one aqueous hydrolysing liquid comprising saccharification enzymes, under conditions in which at least a part of the pretreated biomass material is hydrolysed to a hydrolysate. The hydrolysate thus comprises fermentable sugars. In a step S30, at least one reducing agent selected from sulfur oxyanions and sulfhydryl reagents is added to the lignocellulosic biomass-based material downstream of the reactor vessel for reducing inhibitory substances for saccharification enzymes and/or fermenting microorganism. In the step S30, the concentration of the reducing agent is adapted based on the temperature of the material where it is added, and/or the reducing agent is added to the material having a temperature of T °C, T °C being more than 50 °C.

For example, the step S30 may be performed by that the at least one reducing agent is added to the pretreatment slurry discharged from the reactor vessel, or to the pretreated slurry discharged from the pretreatment arrangement. Such pretreatment slurry, or pretreated slurry, will typically have an elevated temperature of at least 50 °C, which is beneficial as the concentration of the reducing agent can be kept relatively low. The at least one reducing agent may preferably be selected from sulfite, dithionite and dithiothreitol.

As described earlier in this disclosure, for embodiments in which the concentration of the reducing agent is adapted based on the temperature of the material where it is added, the concentration of the reducing agent may be between 5 and 50 mM and the temperature of the material may be between 25 °C and 140 °C. For example, the concentration of the reducing agent may be between 5 and 40 mM and the temperature of the material may be between 40 °C and 140 °C, or the concentration of the reducing agent may be between 5 and 30 mM and the temperature of the material may be between 50 °C and 140 °C. According to one example embodiment, the concentration of the reducing agent is between 12.5 to 50 mM in the temperature range of the material between 25 °C to 50 °C, and between 8.5 to 25 mM in the temperature range of the material between 50 °C and 75 °C, and between 5 to 15 mM in the temperature range between 75 °C and 140 °C. Alternatively, the concentration of the reducing agent is determined based on the equation C(t)=-k^{∗}t + (Const+/- 30%), wherein k and Const are positive numbers, C(t) is the temperature dependent concentration of the reducing agent and t is the temperature in Celsius (°C) of the material at the point of addition of the reducing agent. The term material is here referring to the lignocellulosic biomass-based material at the point of addition of the reducing agent.

According to at least one alternative example embodiment, for embodiments in which the reducing agent is added to the material having a temperature of T °C, T °C is at least 50 °C, or at least 75 °C, or at least 80 °C, or at least 95 °C, or at least 110 °C, and at most 140 °C. Thus, for such embodiments, the concentration of the reducing agent is not necessarily (but it may according to one embodiment) adapted based on the temperature of the material where it is added. Thus, the concentration of the reducing agent may be set to be constant regardless of the temperature of the material at the point of addition of the reducing agent, as long as the temperature is T °C, T °C being at least above 50 °C.

In an optional step S15, preferably performed between the step of pretreating S10 and the step of hydrolysis S20, the pretreated biomass material is separated into a liquid fraction and a solid fraction. Thus, the step S30 may be performed such that the at least one reducing agent is added to the liquid fraction and/or the solid fraction or a suspension thereof.

In an optional step S40 the hydrolysate with the fermentable sugars provided by step S20 is subjected to fermentation in an aqueous liquid utilizing at least one fermenting microorganism under conditions in which at least a part of the fermentable sugars is fermented into a primary target chemical. The steps S20 and S40 may be performed separately from the each other as descried here, or wherein the steps S20 and the step S40 may be performed simultaneously in a single unit. The primary target chemical produced in step S40 may be a fermentation product selected from the group consisting of alcohol, organic acid or aliphatic acid.

The method is preferably automated and adapted for continuous operation. In embodiments, step S30 comprises monitoring the temperature in a continuous or semi-continuous manner and automatically adjusting the concentration of the at least one reducing agent in response to the monitored temperature.For example, the control unit 102 of Fig. 2 may be configured to perform one or more of the method steps described with reference to Fig. 3.

Thus, the control unit 102 may be configured to at least determined the temperature of the material at the point of addition of the reducing agent, and be configured to adjust the concentration of the reducing agent added by the feeding device 170 based on the determined temperature.

As shown in the following Examples, the beneficial effects of addition of at least one reducing agent for which the concentration is adapted to the lignocellulosic biomass-based material at the point of addition of the reducing agent is surprising in at least that the reduction of the inhibitory substances, for at least some temperature intervals, is improved for a lower concentration of the reducing agent compared to a higher concentration. Without desiring to be limited to any specific theory, the results suggest that the reducing agents work better in e.g. protecting the enzymes and/or microorganisms from compounds present in the lignocellulosic biomass-based material downstream of the reactor vessel, at lower concentrations if the temperature is higher. That is, given a range of concentrations of the reducing agent proved to be useful to reduce inhibitory substances, if increasing the temperature at which the reducing agent is added, a lower concentration of the reducing agent is favourable over a higher concentration of the reducing agent, both the lower and higher concentrations being within the proved given range of concentrations. Moreover, addition of reducing agent after the reactor vessel of the pretreatment is beneficial for enzymatic hydrolysis of the pretreated slurry and/or fermentation of the hydrolysate, as the use of residual heat from the reactor vessel enables the use of a relatively low concentration of the reducing agent (moreover, this is regardless of whether sulfur dioxide/sulfite has been used in the pretreatment process).

### Examples

Pretreatment of lignocellulosic biomass material, sawdust of Norway spruce, was performed by Sekab E-Technology in the Biorefinary Demo Plant (BDP), Örnsköldsvik, Sweden. The lignocellulosic biomass material was pretreated using steam explosion in continuous mode and sulfuric acid as impregnating agent. The sulfuric acid charge was 1% (w/w) per dry weight biomass, the temperature was 210 °C, and the residence time was 7-8 min. After the pretreatment of the lignocellulosic biomass material, the pH of the pretreated biomass slurry was approx. 1.5 and the fraction of WIS (water-insoluble solids) was 17.5%.

Before enzymatic saccharification of the samples, the pH of the slurry was adjusted to 5.5 using a 10 M solution of NaOH. The enzyme preparation Cellic CTec 2 was added to the mixture (14 g of liquid enzyme preparation was added to 750 g of slurry), which was then incubated at 50 °C and 140 rpm in an orbital shaker for 48 h. After enzymatic saccharification, the liquid phase, the hydrolysate, was separated from the solid residue by vacuum filtration.

Hydrolysate samples were distributed to reagent bottles with screw caps. A reducing agent was added to each sample, and the samples in each of four series were different based on different concentration of the added reducing agent. For all series, sodium dithionite (Na₂S₂O₄) (Merck, Darmstadt, Germany) was used and added to a final concentration of 5, 8.7 and 12.5 mM, respectively. Moreover, the four series were different based on different prevailing temperatures of the samples, as shown in Table 1. Incubation at higher temperatures (90 °C and 110 °C) was performed using an oil bath. The sodium dithionite was added after the desired temperature had been reached. Magnetic stirring was used, and the treatment time was 10 min.

**Table 1**

| Serie | Sample | Reducing agent conc. | Temperature |
|---|---|---|---|
| 1 | 1a | 5 mM | 50 °C |
| | 1b | 8.7 mM | 50 °C |
| | 1c | 12.5 mM | 50 °C |
| 2 | 2a | 5 mM | 75 °C |
| | 2b | 8.7 mM | 75 °C |
| | 2c | 12.5 mM | 75 °C |
| 3 | 3a | 5 mM | 95 °C |
| | 3b | 8.7 mM | 95 °C |
| | 3c | 12.5 mM | 95 °C |
| 4 | 4a | 5 mM | 110 °C |
| | 4b | 8.7 mM | 110 °C |
| | 4c | 12.5 mM | 110 °C |

Fermentation experiments were conducted using duplicates for each sample in the four series. The fermentation was carried out using 30 ml glass serum bottles containing 25 ml culture medium. The serum bottles were equipped with magnetic stirrer bars and rubber plugs pierced with cannulas for letting out carbon dioxide. The culture medium consisted of 23.5 ml hydrolysate (pH 5.5), 0.5 ml nutrient solution (consisting of 150 g/l yeast extract, 75 g/l (NH₄)₂HPO₄, 3.75 g/l MgSO₄·7 H₂O, and 238.2 g/lNaH₂PO₄-H₂O), and 1.0 ml Saccharomyces cerevisiae yeast inoculum (resulting in 2 g yeast (dry weight) per 1 of culture medium). In addition to treated hydrolysate, reference fermentations containing 23.5 ml of a synthetic glucose solution (97 g/l), 0.5 ml of the nutrient solution, and 1.0 ml of the yeast inoculum were included in the experiment. The bottles were incubated for 60 h at 30 °C with magnetic stirring. Samples for analysis were withdrawn in the beginning of the experiment and after 12, 24, 36, 48 and 60 h of fermentation.

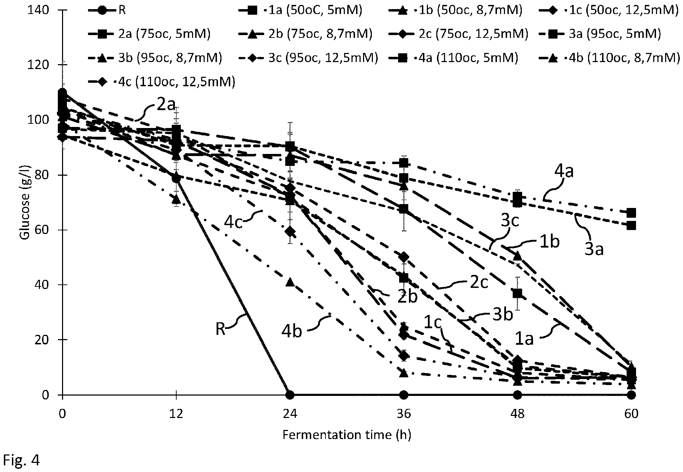

The concentrations of glucose and ethanol in samples withdrawn from the cultures were measured using HPLC (an Agilent 1260 Infinity system equipped with a refractive index detector and a Bio-Rad Aminex HPX-87H column). The results of the glucose measurements are shown in the graph of Fig. 4, which also comprises data of the reference sample having no inhibitory substances (the lines representing the different series are indicated in Fig. 4, and clarified in the following: reference sample R: solid line; serie 1 at 50 °C: long dashes; serie 2 at 75 °C: medium-length dashes; serie 3 at 95 °C: short dashes; serie 4 at 110 °C: alternating medium-length and short dashes; Indicators: reference sample R: filled circles; 5 mM (reducing agent concentration): filled squares; 8.7 mM: filled triangles; 12.5 mM: filled diamonds).

The effects of different concentrations of the reducing agent, and its correlation to the temperature of the sample, are clearly shown in Fig. 4 showing the fermentation based on glucose consumption as a function of the fermentation time. For example, for the samples in series 1 (i.e. at 50 °C), the sample with a reducing agent concentration of 12.5 mM was performing better than the samples with a lower reducing concentration. However, for the other series (i.e. at 75 °C, 85 °C and 110 °C) a relatively lower concentration of the reducing agent gave a better result than a relatively higher concentration of the reducing agent, as the sample with a reducing concentration of 8.7 mM in the respective series had the fastest glucose consumption. Thus, increasing the temperature had a positive effect on the fermentation together with a surprisingly lower concentration of reducing agents.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for reducing inhibitory substances of a lignocellulosic biomass-based material, comprising:
pretreating a lignocellulosic biomass material in a pretreatment arrangement, the pretreating including subjecting the lignocellulosic biomass material to an elevated temperature and pressure in a reactor vessel of the pretreatment arrangement to provide a pretreated biomass material comprising inhibitory substances;
hydrolysing the pretreated biomass material in a hydrolysis unit with at least one aqueous hydrolysing liquid comprising saccharification enzymes, under conditions in which at least a part of the pretreated biomass material is hydrolysed to a hydrolysate, said hydrolysate comprising fermentable sugars;
adding at least one reducing agent selected from sulfur oxyanions and sulfhydryl reagents to the lignocellulosic biomass-based material downstream of the reactor vessel for reducing inhibitory substances related to saccharification enzymes and/or fermenting microorganism, wherein the concentration of the reducing agent is adapted based on the temperature of the material where it is added, and/or wherein the reducing agent is added to the material having a temperature of T °C, T °C being more than 50 °C.

2. The method according to claim 1, wherein the pretreated biomass material is discharged from the reactor vessel as a pretreatment slurry having an elevated temperature of at least 50 °C, and wherein the at least one reducing agent is added to the pretreatment slurry at the elevated temperature.

3. The method according to any one of the preceding claims, in which the concentration of the reducing agent is adapted based on the temperature of the material where it is added, and wherein the concentration of the reducing agent is between 5 and 50 mM and the temperature of the material is between 25 °C and 140 °C, or wherein the concentration of the reducing agent is between 5 and 40 mM and the temperature of the material is between 40 °C and 140 °C, or wherein the concentration of the reducing agent is between 5 and 30 mM and the temperature of the material is between 50 °C and 140 °C.

4. The method according to any one of the preceding claims, in which the concentration of the reducing agent is adapted based on the temperature of the material where it is added, and wherein the concentration of the reducing agent:
is between 12.5 to 50 mM in the temperature range of the material between 25 °C to 50 °C, and between 8.5 to 25 mM in the temperature range of the material between 50 °C and 75 °C, and between 5 to 15 mM in the temperature range between 75 °C and 140 °C.

5. The method according to any one of the preceding claims, in which the concentration of the reducing agent is adapted based on the temperature t of the material where it is added, and wherein the concentration C(t) of the reducing agent:
is determined based on the equation C(t)=-k^{∗}t + (Const+/- 30%), wherein k and Const are positive numbers.

6. The method according to any one of the preceding claims, in which the reducing agent is added to the material having a temperature of more than T °C, and wherein T is at least 50 °C, or at least 75 °C, or at least 80 °C, or at least 95 °C, or at least 110 °C, and at most 140 °C.

7. The method according to any one of the preceding claims, further comprising separating the pretreated biomass material into a liquid fraction and a solid fraction, wherein the at least one reducing agent is added to the liquid fraction and/or the solid fraction or a suspension thereof.

8. The method according to any one of the preceding claims, comprising subjecting the hydrolysate with the fermentable sugars to fermentation in an aqueous liquid utilizing at least one fermenting microorganism under conditions in which at least a part of the fermentable sugars is fermented into a primary target chemical.

9. The method according to any one of the preceding claims, wherein the primary target chemical is a fermentation product selected from the group consisting of alcohol, organic acid or aliphatic acid

10. The method according to any one of the preceding claims, wherein the at least one reducing agent is selected from sulfite, dithionite and dithiothreitol.

11. Use of at least one reducing agent for decreasing the enzymatic hydrolysis inhibitory properties, and/or decreasing the microorganisms fermentation inhibitory properties of a pretreated lignocellulosic biomass-based material by adapting the concentration of the reducing agent based on the temperature of the material where it is added, and/or by adding the reducing agent to the material having a temperature of more than T °C, T being more than 50 °C.

12. A system for reducing inhibitory substances of a lignocellulosic biomass-based material, comprising:
a pretreatment arrangement for pretreating a lignocellulosic biomass material, the pretreatment arrangement comprises a reactor vessel configured to subject the lignocellulosic biomass material to an elevated temperature and pressure to provide a pretreated biomass material comprising inhibitory substances;
a hydrolysing unit configured to treat the pretreated biomass material with at least one aqueous hydrolysing liquid comprising saccharification enzymes under conditions in which at least a part of the pretreated biomass material is hydrolysed to a hydrolysate comprising fermentable sugars;
a feeding device configured to add at least one reducing agent selected from sulfur oxyanions and sulfhydryl reagents to the lignocellulosic biomass-based material downstream of the reactor vessel for reducing inhibitory substances related to saccharification enzymes and/or fermenting microorganism, wherein the feeding device is configured to adapt the concentration of the reducing agent based on the temperature of the material where it is added, and/or wherein the feeding device is configured to add the reducing agent to the material having a temperature of T °C, T °C being more than 50 °C.

13. The system according to claim 12, further comprising means for subjecting the hydrolysate with the fermentable sugars to fermentation in an aqueous liquid utilizing at least one fermenting microorganism under conditions in which at least a part of the fermentable sugars is fermented into a primary target chemical.

14. The system according to any one of claims 12-13, further comprises a measuring device configured to measure the temperature of the lignocellulosic biomass-based material downstream of the reactor vessel, and at the point of addition of the at least one reducing agent.
